# EUROPEAN PATENT SPECIFICATION

(11) **EP 2 836 581 B1**
(45) Date of publication and mention of the grant of the patent: **07.04.2021**
(21) Application number: 13706882.1
(22) Date of filing: 15.02.2013
(51) Int. Cl.: C11D 3/22, A61K 8/73

(54) **A HIGHLY EFFICIENT AND CONVENIENT FORM OF MICROFIBROUS CELLULOSE**
HOCHEFFIZIENTE UND ZWECKMÄSSIGE FORM VON MIKROFASERCELLULOSE
FORME DE CELLULOSE MICROFIBREUSE TRÈS EFFICACE ET COMMODE

(30) Priority: 13.04.2012 US 201261624086 P
(43) Date of publication of application: 18.02.2015
(73) Proprietor: CP Kelco U.S., Inc., Atlanta, GA 30339 (US)
(72) Inventor: SWAZEY, John, San Diego, CA 92130 (US); MORRISON, Neil, San Diego, CA 92128 (US); YANG, Zhi-Fa, San Diego, CA 92129 (US); COMPTON, Jacqueline, San Diego, CA 92101 (US); NOLAN, Tim, Chula Vista, CA 91915 (US)
(74) Representative: Murgitroyd & Company
(86) International application number: PCT/US2013/026355
(87) International publication number: WO 2013/154675

(56) References cited:
- US-A1- 2008 108 714
- US-A1- 2010 016 575

## Description

### BACKGROUND

Viscosity modifiers are used in a variety of products-from foods, pharmaceuticals, and cosmetics to oil field drilling fluids. One such viscosity modifier is microfibrous cellulose (MFC), also known as reticulated cellulose or as microfibrillated cellulose, which may be produced by fermentation of *Acetobacter xylinum.* This bacteria produces cellulose that is chemically identical to plant-derived cellulose. Though identical in chemical structure, MFC fibers may be smaller in diameter than plant-derived cellulose fibers, thereby giving MFC a greater surface area. This high surface area allows MFC to create three-dimensional networks that produce a desirable yield value in solution at low use levels. MFC is essentially insoluble and uncharged and, therefore, may not be not adversely affected by ionic environments. Because MFC is essentially insoluble it does not compete for water and, therefore, has a wide range of compatibility and is much less susceptible to degradation than water-soluble polysaccharides. It is compatible with both concentrated anionic aqueous solutions, such as heavy brines used in oilfield applications, and in high surfactants systems, such as liquid dish and laundry detergents (see, e.g., U.S. Published Patent Applications 2008/0108541 A1; 2008/0108714 A1). MFC is also compatible with cationic systems, such as fabric softeners using cationic softening agents and anti-microbial cleaners that use benzylalkonium chlorides (see, e.g., U.S. Patent 6,241,812; U.S. Patent 7,888,308). It also can be used in polyol systems (see, e.g., U.S. Patent 5,951,910) such as in essentially pure glycerin, ethylene glycol, propylene glycol, and polyethylene glycol systems.

MFC has been produced commercially in several forms. One form of MFC that was produced commercially for several years by CP Kelco, but later discontinued, was as a wet cake (resembling wet cardboard). This form of MFC was typically 10 - 20 wt% solids and the balance water. A small amount of sorbic acid preservative was as added to prevent mold. This form of MFC was processed using multiple rinse cycles, which led to significant product loss in recovery, and also included strong alkali treatment, which appears to have led to a decrease in MFC efficiency.

Dry powder forms of MFC are commercially available, including discontinued products such as PrimaCel™ and more recent products such as AxCel® PX, AxCel® CG-PX, Axcel® PG, Cellulon® PX, and various "K"-namedproducts (CP Kelco U.S., Inc., Atlanta, GA). Dry powder forms were created to improve handling and logistics for delivery to customers (such as issues and cost associated with transporting water). These commercial versions of powdered MFC can be used to provide suspension in many applications, such as surfactant-thickened and high surfactant systems (see, e.g., U.S. Published Patent Application Nos. 2008/0108541, 2008/0108714, and U.S. Patent 7,888,308. These commercial versions of powdered MFC comprise a blend of MFC and various co-agents, such as, but not limited to, carboxymethyl cellulose (CMC), xanthan gum, guar, pectin, gellan, carrageenan, locust bean gum, gum Arabic, cationic guar, cationic hydroxyethyl cellulose (HEC). Additional information regarding MFC systems can be found, for example, in U.S. Published Patent Application No. 2007/0027108 and U.S. Patent 8,053,216.

These co-agents allow the drying and milling of MFC into a powdered product. Without these co-agents, MFC loses a high degree of its functionality after drying and milling due to an irreversible agglomeration of the MFC during the drying process known as hornification. Blends with co-agents, however, may limit how powdered MFC can be used in products due to compatibility limitations of the co-agents. For example, while MFC is uncharged, most of the co-agents that are used are either anionic or cationic. Thus, commercial MFC products containing anionic co-agents, such as CMC or xanthan gum, may have compatibility issues when used in products with, for instance, cationic surfactants. Additionally, commercial powder MFC may have limited compatibility with products that contain high levels of water-miscible organic solvents, such as glycols or glycerol. When used with such organic solvents, the co-agents from the commercial MFC may form precipitates which may result in poor clarity and poor yield values (i.e., poor suspension properties). Finally, the use of activated solutions (i.e., highly dispersed solutions of MFC prepared by using high shear mixing such as high speed rotor stator devices or high pressure homogenization such as the Ross Quad/Megashear or APV Gaulin) of powdered MFC may restrict the order in which other ingredients are added to a product formulation, so as to prevent issues such as co-agents forming precipitates.
Accordingly, there exists a need to provide a form of MFC product for use in a wide variety of product formulations that increases the efficiency of the MFC over current commercially available forms of MFC. US2010/016575A1 discloses a bacterial cellulose-containing formulation and a method for the production of said bacterial cellulose-containing formulation.

### SUMMARY

Disclosed herein is a composition comprising micro fibrous cellulose broth and surfactant which has an improved yield value as compared to powdered MFC when used at the same concentration as the micro fibrous cellulose broth in accordance with the claims.

Also disclosed is a composition comprising an effective amount of microfibrous cellulose broth and an effective amount of surfactant, the composition having a yield value that is higher than the same composition comprising wet cake MFC at the same concentration as the microfibrous cellulose broth in accordance with the claims.

Further disclosed is a composition with an increased yield value relative to the same composition comprising wet cake MFC or powder MFC, the composition comprising an effective amount of microfibrous cellulose broth and surfactant in accordance with the claims.

### DETAILED DESCRIPTION

Before the present methods are disclosed and described, it is to be understood that the aspects described below are not limited to specific embodiments, specific embodiments as such may, of course, vary. It is also to be understood that the terminology used herein is for the purpose of describing particular aspects only and is not intended to be limiting.

It must be noted that, as used in the specification and the appended claims, the singular forms "a," "an," and "the" include plural references unless the context clearly dictates otherwise. Thus, for example, reference to "a co-agent" include mixtures of two or more such co-agents or references to a "bleaching" or "oxidizing agent" includes mixtures of two or more such agents.

A weight percent of a component, unless specifically stated to the contrary, is based on the total weight of the formulation or composition in which the component is included.

It has been found that MFC in its broth form is particularly convenient and efficient relative to other forms of MFC. The broth can either be used "as is" directly from the fermentor or can undergo further processing steps to deodorize and sterilize the broth. Broth, as used herein, does not refer to a wet cake or a powder form MFC. Rather, broth, as used herein, is intended to include fermentation media comprising a usable amount of microbially produced MFC. Broth also includes fermentation media that is further processed so long as the character of the MFC is not changed so much as to lose its original efficiency (as apparently takes place during alkali treatment at pH 13 and 65.56°C and after several dewatering steps on a belt press). For example, a polished fermentation media that still has a 20%, 30%, 40%, or higher increase in yield stress compared to wet-cake MFC is within this scope of the broth form.

In embodiments, deodorizing and sterilizing of the broth can be done by using appropriate oxidizing agents, such as hydrogen peroxide and/or sodium hypochlorite, or by using pasteurization techniques with or without chemical treatments. Treatment can also be done to whiten and deodorize the fermented solution, for example, by using bleaching agents or by diluting or rinsing the MFC broth. Typical amounts of such agents are 0.01 - 1 wt% (for example, good deoderization and whiting was obtained using 0.25 wt% active hydrogen peroxide without measurable losses in performance compared to the untreated broth). Higher levels of treatment may be possible without affecting performance, but are not typically needed to have the desirable deodorization and whitening without appreciable residual oxidants remaining. One of skill in the art can determine appropriate agents, concentrations and reaction conditions which will can achieve the desired result but not adversely affect the efficiency of the MFC.

Also, treatment can be done to concentrate the MFC in the broth using, for example, decanting centrifuges.

An additional benefit of the broth form of MFC is that the broth can be pre-activated, e.g., passed through high shear mixers such multi-stage rotor-stator devices or high pressure homogenizers, to yield a solution of fully activated MFC that can be added directly to a wide range of formulations to impart the desired yield value (i.e., suspensional properties) without typically requiring the use of additional high shear mixing by the end user.

In embodiments, co-agents can be added to the MFC broth if desired. For example, carboxymethyl cellulose (CMC) is known as an effective co-agent to help prevent flocculation of MFC in high water systems. Also, cationic guar and cationic HEC can be used in the same manner as CMC to prevent flocculation of MFC in high water systems where these polymers are soluble. These cationic polymers can help prevent MFC flocculation in applications such as fabric softeners, hair and skin conditioners, and anti-bacterial hand cleaners and household cleaners that use quaternary amine-based surfactants. Also, there is some evidence that optical brighteners commonly found in laundry detergents can serve as effective co-agents to prevent MFC flocculation, such as CAL Fluor® dyes. Other co-agents that may show more modest benefits to MFC stabilization (by prevention of MFC flocculation) include xanthan gum, diutan gum, welan gum, scleroglucan gum, succinoglucan gum, gellan gum, guar gum, pectin, carrageenan, locust bean gum, gum arabic, hydroxyethyl cellulose, hydroxypropyl cellulose, methylcellulose, carbomer, and non-ionic thickening surfactants such as some Pluronic® surfactants. Example amounts of co-agents are as little 1 part co-agent to 10 parts MFC for those co-agents such as CMC, cationic guar, and cationic HEC which adsorb onto the fibers and provide a repelling charge to use levels of 0.25 - 1 wt% or more for co-agent that merely provide a viscous matrix that prevents or slows the migration of the fibers together in a formulation.

It has been discovered that by using an MFC fermentation broth, or polished versions thereof, the MFC is highly efficient. For example in our particular Example embodiments, the MFC provided an average of 40% improvement in yield values over previously available commercial forms of MFC. For example, use levels of only 0.024% active MFC broth can provide over 1 Pa of yield value when present in many surfactant formulations.

This efficiency benefit of MFC broth allows for preparation of higher clarity formulations due to the lower amount of MFC broth required as compared to previously available forms of MFC, since MFC tends to cloud formulations as concentrations rise.

Example applications where MFC may be useful include providing suspension properties to high surfactant systems like liquid dish and liquid laundry detergent, personal care formulations including liquid bodywash, liquid hand wash, shampoos, and conditioners. The non-ionic nature of the MFC makes it equally useful in cationic formulations such as liquid fabric softeners, conditioning skin lotion and hair conditioners, and anti-microbial formulations using quaternary amine surfactants. In some of the examples above, the addition of a co-agent may provide extra stability to the MFC to prevent flocculation of the MFC fibers that may lead to more hazing and possible destabilization of the suspension properties of the MFC.

### Method for Providing a Highly Efficient and Convenient Form of MFC

One of skill in the art can use known conventional methods of fermenting *A. xylinum.* No special media, temperature or pressure are required to produce the broth of the present invention. A fermentation broth of *Acetobacter xylinum* typically produces microfibrous cellulose at levels from 0.5 to 2.0 g/liter. The remainder of the broth is predominately water with low levels of residual sugars, proteins, cellular debris, and a trace amount of a variety of salts used to provide the bacteria its required nutrients and pH control. In many end use industrial applications for the MFC, such side products have no influence of the final application, and so the use of MFC directly from the broth poses no significant problems. It is often required to kill any active micro-organisms at the end of the fermentation cycle, and this can be done by pasteurization at the proper temperature and/or chemical treatment. Numerous methods can be used to "polish" the MFC broth, if desired. Polishing primarily improve the aesthetics of the MFC broth rather than changing its performance. For example, chemical bleaches and peroxides can be used to both deodorize and whiten the MFC broth.

Decanting centrifuges can be used to concentrate the MFC in the broth or concentrate the broth after washing steps without diminishing the MFC concentration in the final broth product due to product losses that are typically experienced with belt presses. Dilution of the broth can also be used to provide a more fluid product or one that can be more easily activated due to its improved flow properties. In the more dilute ranges, a co-agent such as CMC, cationic guar, or cationic HEC can be used to improve the homogeneity of the broth or to provide additional dispersion of the MFC fibers in lower surfactant applications like bodywash, shampoo, and liquid handwash. Typical ranges of MFC concentration in the broth range from 0.5 wt% MFC to over 10 wt% MFC, if concentration methods are used. Other suitable methods also may be used to concentrate the MFC in the broth or concentrate the broth after washing steps, including sedimentation or settling tanks, evaporators, or any other method or device which is capable of maintaining the fiber distribution of the MFC and which does not substantially impair the functionality of the MFC.

### Method for Making a Product Formulation Using MFC

The compositions of MFC broth can be used in a variety of product formulations and applications. MFC broth, for example, can be effectively used in cationic fabric softeners and cationic cleaners. By contrast, solutions prepared from commercially available powdered MFC will react strongly with these cationic systems and lead to strong precipitation due to the anionic co-agent present in the powdered MFC product. MFC broth also can be effectively used in neutral and anionic compositions.

MFC broth can be used in high salt systems including saturated salt systems.

Also, these broth MFC solutions can work effectively to thicken or provide suspension in polyol compositions, e.g., PEG 300, glycerine, ethylene glycol, and propylene glycol solutions, and some alcohol systems. Advantageously, this can be done using only the water contributed by the aqueous broth solutions of MFC as it is incorporated.

Additionally, broth MFC solutions have a relative insensitivity to order of addition into high surfactant systems, whereas solutions prepared from powdered MFC show significant sensitivity to order of addition. This is because of the co-agent sensitivity due to incompatibility with many high surfactant and surfactant-thickened formulations.

The compositions and the methods disclosed hereinabove can be used to make numerous personal care products, e.g., bodywashes, hand soaps, and shampoos that incorporate the smooth, rich, thickening properties imparted by surfactant-thickening agents, but also having the ability to suspend matter due to the higher yield imparted by the broth MFC. Also, the compositions and the methods of this disclosure can be used to make dishwashing soap with suspended actives (e.g., moisturizing beads) or decorative items or laundry detergents with suspended actives, such as insoluble enzymes, encapsulated actives, and zeolites. The compositions and the methods of this disclosure can also be useful with cationic systems like fabric softeners, anti-microbial cleaners, skin lotions, and hair conditioners containing cationic surfactants. Finally, the compositions and the methods of this disclosure can be useful to provide suspension to nonaqueous systems like PEG solutions used as carrier fluids to suspend hydrocolloids or other particulate material.

Use levels of the MFC in these applications can range from 0.001 wt% to 0.25 wt% active MFC, with more typical use levels in the 0.015 - 0.06 wt% active MFC range.

Activation is easier with broth than the other forms of MFC -- for example, to prepare a 3 wt% concentration composition of AxCel® PX powder MFC (60% MFC), activation at 3000 psi requires two passes through a pressure homogenizer, whereas the broth form shows good activation at 1500 psi in one pass with up to 2.0 wt% MFC.

The present disclosure is further illustrated by the following examples, which are not to be construed in any way as imposing limitations upon the scope thereof.

### EXAMPLES

### Example 1: MFC broth addition to provide suspension in a commercial dish soap.

Step 1 : Activate broth MFC solution by using any of several example methods:

Lab Scale: 200 g of broth in a 200 g closed Oster puree mixing jar and mix on an Oster blender at top speed for 5 minutes. This method can typically be used to activate MFC broth solutions with up to 1% active MFC.

Lab Scale: 1000 g of MFC broth in a 2 liter plastic beaker and mix on a Silverson L4RT- A equipped with the emulsion screen. Mix for 10 minutes at 10,000 rpm with constant movement of the beaker. This method can typically be used to activate MFC broth solutions with up to 1% active MFC.

Lab Scale to Plant Scale: Activate MFC broth using an APV Gaulin pressure dual stage homogenizer. Activation can be done up to 1.5% MFC and higher at sufficient pressures and passes. For example, at 0.6%-2.0% active MFC broth, activation can be in one pass at as little as 13.8 MPa (2000 psi). For the examples listed in the tables below, the Oster puree mixing jar was used.

Step 2: Weigh out the desired amount of Palmolive Ultra Green Apple Dish Detergent into an 8 oz. jar. Add a sufficient amount of activated MFC broth to the jar containing the dish soap to result in a 0.024% or 0.0336% active MFC.

For the example table below, a 0.6% active MFC diluted broth was used. 8 g of this activated MFC broth solution was used along with 192 g of Palmolive Ultra dish soap (Colgate-Palmolive

Company, New York, NY) to result in a 200 g final solution at 0.024% active MFC. Also, 11 g of this activated MFC broth solution was used along with 189 g of Palmolive Ultra dish soap to result in a 200 g final solution at 0.0336% active MFC. The solution was then mixed for about 5 minutes at 800 rpm using a jiffy mixing blade. The resulting solution was then completely deaerated using a centrifuge at about 150 gs, and the yield was measured using a Brookfield Ultra RV with a #71 vane spindle.

The results of the broth MFC were compared to a comparable level of active MFC from an MFC powdered product. The results in Table 1 show the superior performance of a number of unique broth MFCs over a typical powdered MFC in Palmolive Ultra Dish Soap. Table 2 shows that this superior efficiency is significant even at higher use levels of MFC.

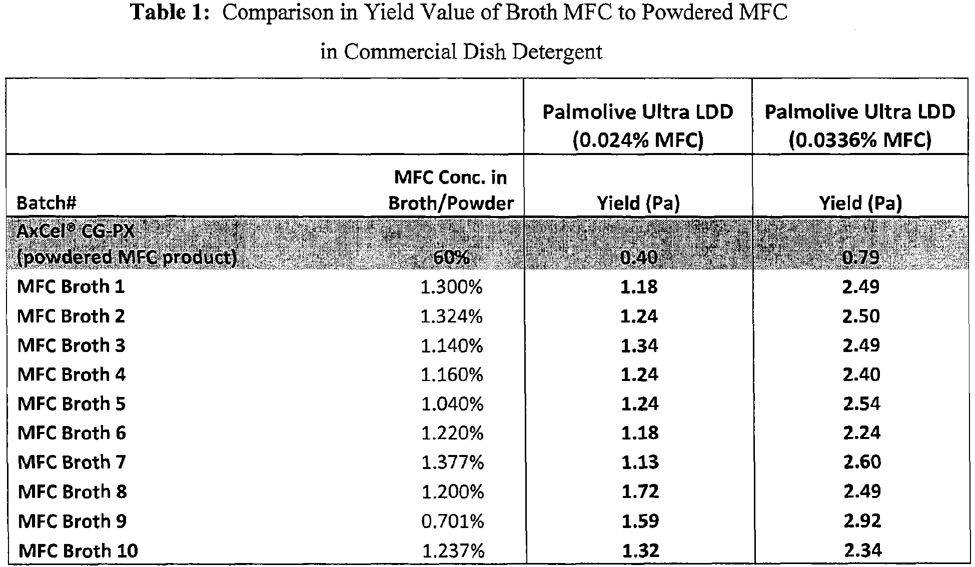

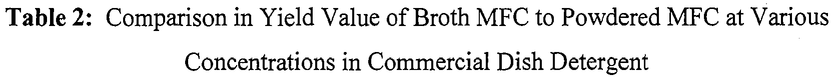

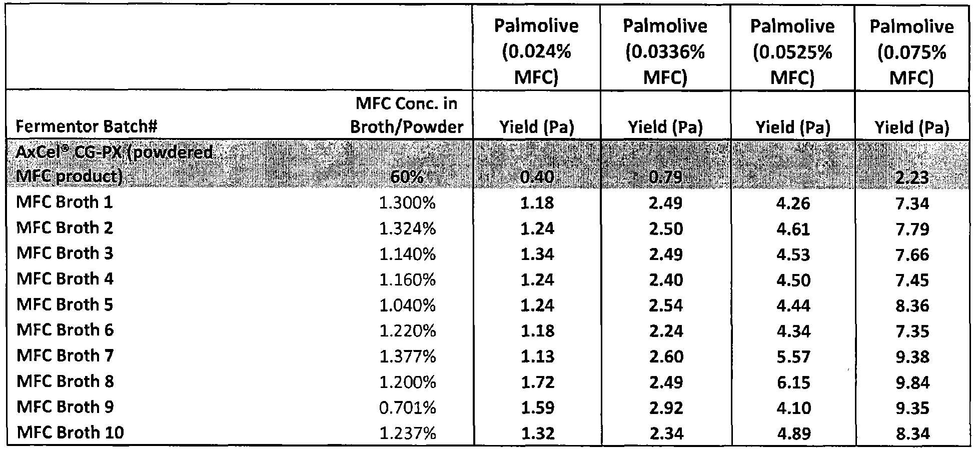

### Example 2: MFC broth addition to provide suspension in a commercial, liquid laundry detergent.

Step 1: Activate MFC broth solution using the method detailed in Example 1.
Step 2: Weigh out 189 g of Tide HE 2x Free and Clear liquid laundry detergent (Procter & Gamble, Cincinnati, OH) into an 0.241 (8 oz.) jar. Add a sufficient amount of activated MFC broth to the jar containing the dish soap to result in a 0.024% active MFC.

For the example table below, a 0.6% active MFC diluted broth was used. 11 g of this activated MFC broth solution was used with 189 g of Tide HE 2x Free and Clear LLD to result in a 200 g final solution at 0.0336% active MFC. The solution was then mixed for about 5 minutes at 800 rpm using a jiffy mixing blade. The resulting solution was then completely deaerated using a centrifuge at about 150 gs, and the yield was measured using a Brookfield Ultra RV with a #71 vane spindle.

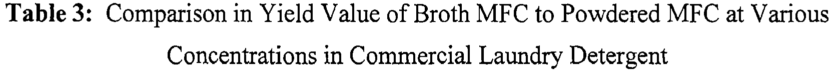

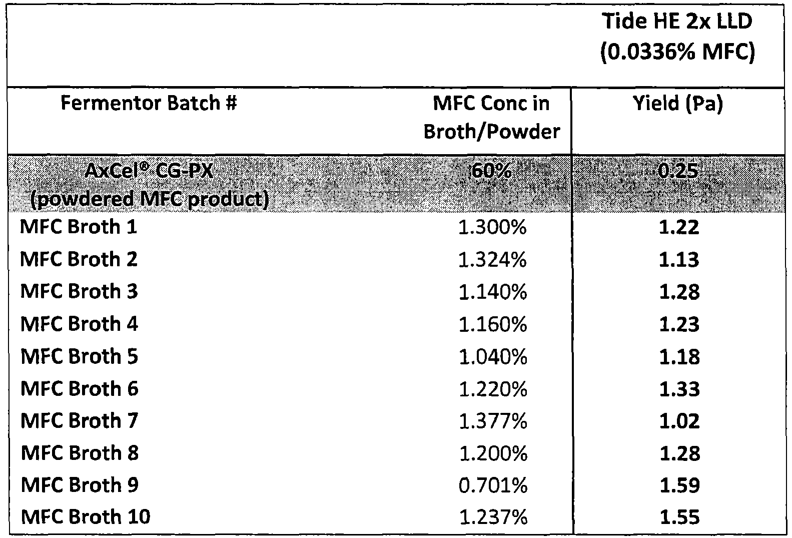

MFC provided as a broth was also surprisingly found to have higher efficiency than the wet-cake form of MFC. The reason for this is not fully known, but it may be due to some of the limitations of wet-cake recovery and dewatering by belt press which can lead to the loss of some fractions of the highest surface area fibers of the microfibrous cellulose. Testing of 5 lots of MFC wet-cake gave an average of 0.97 Pa yield value as measured in a Tide HE 2x liquid laundry detergent at 0.0336% active MFC, whereas the results of 20 broth batches gave an average of 1.36 Pa yield value. A table of results is given below:

**Table 4: Comparison in Yield Value of Broth MFC to Wet Cake MFC at Various Concentrations in Commercial Laundry Detergent**

| **Fermentation Batch#** | **MFC Concentration** | **Tide HE 2x (0.0336% MFC)(Pa)** RV, #71, Secondary Immersion **Yield (Pa)** |
|---|---|---|
| | | |
| **MFC broth 1** | 1.300% | **1.22** |
| **MFC broth 2** | 1.324% | **1.13** |
| **MFC broth 3** | 1.140% | **1.28** |
| **MFC broth 4** | 1.160% | **1.23** |
| **MFC broth 5** | 1.040% | **1.18** |
| **MFC broth 6** | 1.220% | **1.33** |
| **MFC broth 7** | 1.377% | **1.02** |
| **MFC broth 8** | 1.200% | **1.28** |
| **MFC broth 9** | 0.701% | **1.59** |
| **MFC broth 10** | 1.237% | **1.55** |
| **MFC broth 11** | 1.256% | **1.29** |
| **MFC broth 12** | 1.247% | **1.42** |
| **MFC broth 13** | 1.336% | **1.83** |
| **MFC broth 14** | 1.217% | **1.40** |
| **MFC broth 15** | 1.183% | **1.40** |
| **MFC broth 16** | 1.000% | **1.23** |
| **MFC broth 17** | 1.406% | 1.39 |
| **MFC broth 18** | 1.14% | **1.55** |
| **MFC concentrated broth 19** | 2.00% | **1.60** |
| | **Average Broth Yield Value:** | **1.36** |
| **Wet-Cake Form of MFC** | **MFC Conc.** | **Yield (Pa)** |
| **Batch#** | | |
| **wet-cake 1** | 26.5% | **1.30** |
| **wet-cake 2** | 19.2% | **0.66** |
| **wet-cake 3** | 21.2% | **0.78** |
| **wet-cake 4** | 21.2% | **0.88** |
| **wet-cake 5** | 21.1% | **1.25** |
| | **Average Wet-Cake Yield Value:** | **0.97** |

**Table 5: Summary of Tables 1-4.**

| | **Broth** | **Wet cake** | **Powder** |
|---|---|---|---|
| | **Yield (Pa)** | | |
| **Dish soap with** | **Avg.** | | |
| **0.024% MFC** | 1.20 | | 0.40 |
| **0.0336% MFC** | 2.50 | | 0.79 |
| **0.0525% MFC** | 4.74 | | N/A |
| **0.075% MFC** | 8.29 | | 2.23 |
| | | | |
| **Laundry Detergent with** | **Avg.** | **Avg.** | |
| **0.0336% MFC** | 1.36 | 0.97 | 0.25 |

## Claims

1. A composition comprising
a) a microfibrous cellulose broth, and
b) a surfactant
wherein the microfibrous cellulose broth and the surfactant are each present in the composition in an effective amount to produce the composition having a concentration of active microfibrous cellulose, such that the composition has a yield value higher than that of a comparative composition comprising the surfactant and a powdered microfibrous cellulose, a wet cake microfibrous cellulose, or a combination thereof at an amount that imparts to the comparative composition a concentration of active microfibrous cellulose which is the same as the active microfibrous cellulose concentration of the composition.

2. The composition of claim 1, wherein the effective amount of microfibrous cellulose broth is such that the active microfibrous cellulose concentration is from 0.001 to 0.25% by weight of the composition.

3. A method of improving a yield value of a surfactant composition comprising adding a microfibrous cellulose broth to a composition comprising a surfactant wherein the microfibrous cellulose broth and the surfactant are each present in the composition in an effective amount to produce the composition having a concentration of active microfibrous cellulose, such that the composition has a yield value higher than that of a comparative composition comprising the surfactant and a powdered microfibrous cellulose, a wet cake microfibrous cellulose, or a combination thereof at an amount that imparts to the comparative composition a concentration of active microfibrous cellulose which is the same as the active microfibrous cellulose concentration of the composition.

4. The method of claim 3, wherein the effective amount of microfibrous cellulose broth is such that the active microfibrous cellulose concentration is from 0.001 to 0.25% by weight of the composition.

5. The method of claim 3, wherein the composition is
(i) a personal care product selected from the group consisting of bodywashes, hand soaps, shampoos, conditioners, and skin lotions; or
(ii) a cleaning product selected from the group consisting of dishwashing soaps, laundry detergents, fabric softeners, and antimicrobial cleaners.

## Patentansprüche

1. Eine Zusammensetzung, die Folgendes beinhaltet:
a) eine Mikrofasercellulosebrühe, und
b) ein Tensid
wobei die Mikrofasercellulosebrühe und das Tensid jeweils in der Zusammensetzung in einer wirksamen Menge vorhanden sind, um die Zusammensetzung, die eine Konzentration von aktiver Mikrofasercellulose aufweist, zu erzeugen, sodass die Zusammensetzung eine höhere Fließgrenze aufweist als die einer vergleichbaren Zusammensetzung, die das Tensid und eine pulverisierte Mikrofasercellulose, eine Filterkuchen-Mikrofasercellulose oder eine Kombination davon in einer Menge beinhaltet, die der vergleichbaren Zusammensetzung eine Konzentration von aktiver Mikrofasercellulose verleiht, die der Konzentration von aktiver Mikrofasercellulose der Zusammensetzung gleich ist.

2. Zusammensetzung gemäß Anspruch 1, wobei die wirksame Menge von Mikrofasercellulosebrühe derart ist, dass die Konzentration von aktiver Mikrofasercellulose von 0,001 Gew.-% bis 0,25 Gew.-% der Konzentration beträgt.

3. Ein Verfahren zum Verbessern einer Fließgrenze einer Tensidzusammensetzung, das das Hinzugeben einer Mikrofasercellulosebrühe zu einer ein Tensid beinhaltenden Zusammensetzung beinhaltet, wobei die Mikrofasercellulosebrühe und das Tensid jeweils in der Zusammensetzung in einer wirksamen Menge vorhanden sind, um die Zusammensetzung, die eine Konzentration von aktiver Mikrofasercellulose aufweist, zu erzeugen, sodass die Zusammensetzung eine höhere Fließgrenze aufweist als die einer vergleichbaren Zusammensetzung, die das Tensid und eine pulverisierte Mikrofasercellulosebrühe, eine Filterkuchen-Mikrofasercellulose oder eine Kombination davon in einer Menge beinhaltet, die der vergleichbaren Zusammensetzung eine Konzentration von aktiver Mikrofasercellulose verleiht, die der Konzentration von aktiver Mikrofasercellulose der Zusammensetzung gleich ist.

4. Verfahren gemäß Anspruch 3, wobei die wirksame Menge von Mikrofasercellulosebrühe derart ist, dass die Konzentration von aktiver Mikrofasercellulose von 0,001 Gew.-% bis 0,25 Gew.-% der Konzentration beträgt.

5. Verfahren gemäß Anspruch 3, wobei die Zusammensetzung Folgendes ist:
(i) ein Körperpflegeprodukt, ausgewählt aus der Gruppe, bestehend aus Körperwaschmitteln, Handseifen, Haarwaschmitteln, Haarspülungen und Hautlotionen; oder
(ii) ein Reinigungsprodukt, ausgewählt aus der Gruppe, bestehend aus Geschirrspülseifen, Waschmitteln, Weichspülern und antimikrobiellen Reinigern.

## Revendications

1. Une composition comprenant
a) un bouillon de cellulose microfibreuse, et
b) un tensioactif
dans laquelle le bouillon de cellulose microfibreuse et le tensioactif sont chacun présent dans la composition en une quantité efficace pour produire la composition ayant une concentration de cellulose microfibreuse active, de telle sorte que la composition a une valeur de limite d'écoulement plus élevée que celle d'une composition comparative comprenant le tensioactif et une cellulose microfibreuse pulvérulente, une cellulose microfibreuse en pain humide, ou une combinaison de celles-ci à hauteur d'une quantité qui confère à la composition comparative une concentration de cellulose microfibreuse active qui est la même que la concentration de cellulose microfibreuse active de la composition.

2. La composition de la revendication 1, dans laquelle la quantité efficace de bouillon de cellulose microfibreuse est telle que la concentration de cellulose microfibreuse active vaut de 0,001 à 0,25 % en poids de la composition.

3. Un procédé d'amélioration d'une valeur de limite d'écoulement d'une composition de tensioactif comprenant le fait d'ajouter un bouillon de cellulose microfibreuse à une composition comprenant un tensioactif, dans lequel le bouillon de cellulose microfibreuse et le tensioactif sont chacun présent dans la composition en une quantité efficace pour produire la composition ayant une concentration de cellulose microfibreuse active, de telle sorte que la composition a une valeur de limite d'écoulement plus élevée que celle d'une composition comparative comprenant le tensioactif et une cellulose microfibreuse pulvérulente, une cellulose microfibreuse en pain humide, ou une combinaison de celles-ci à hauteur d'une quantité qui confère à la composition comparative une concentration de cellulose microfibreuse active qui est la même que la concentration de cellulose microfibreuse active de la composition.

4. Le procédé de la revendication 3, dans lequel la quantité efficace de bouillon de cellulose microfibreuse est telle que la concentration de cellulose microfibreuse active vaut de 0,001 à 0,25 % en poids de la composition.

5. Le procédé de la revendication 3, dans lequel la composition est
(i) un produit de soins personnels sélectionné dans le groupe constitué des gels douche, des savons pour les mains, des shampooings, des après-shampooings, et des lotions pour la peau ; ou
(ii) un produit de nettoyage sélectionné dans le groupe constitué des savons pour la vaisselle, des détergents pour la lessive, des adoucissants pour le linge, et des nettoyants antimicrobiens.
